# FASCICULE DE BREVET EUROPEEN

(11) **EP 2 580 178 B1**
(45) Date de publication et mention de la délivrance du brevet: **03.09.2014**
(21) Numéro de dépôt: 11728642.7
(22) Date de dépôt: 27.05.2011
(51) Int. Cl.: C07C 5/27, C07C 11/09, C07C 1/24, C07C 11/08

(54) **PROCÉDÉ DE DÉSHYDRATATION ET D'ISOMÉRISATION D'ALCOOLS EN C4 UTILISANT UN SOLIDE AMORPHE À POROSITÉ ADAPTÉE**
VERFAHREN ZUR DEHYDRIERUNG UND ISOMERISIERUNG VON C4-ALKOHOLEN MIT EINEM AMORPHEN FESTSTOFF MIT GEEIGNETER POROSITÄT
PROCESS FOR DEHYDRATION AND ISOMERIZATION OF C4 ALCOHOLS USING AN AMORPHOUS SOLID WITH SUITABLE POROSITY

(30) Priorité: 11.06.2010 FR 1002469
(43) Date de publication de la demande: 17.04.2013
(73) Titulaire: IFP Energies nouvelles, 92852 Rueil-Malmaison Cedex (FR)
(72) Inventeur: COUPARD, Vincent, F-69100 Villeurbanne (FR); MAURY, Sylvie, F-69390 Charly (FR); SURLA, Karine, F-69560 St Cyr sur le Rhône (FR)
(86) Numéro de dépôt international: PCT/FR2011/000316
(87) Numéro de publication internationale: WO 2011/154621

(56) Documents cités:
- EP-A1- 0 071 199
- EP-A1- 0 659 719
- FR-A1- 2 484 400
- MACHO V ET AL: "Dehydration of C4 alkanols conjugated with a positional and skeletal isomerisation of the formed C4 alkenes", APPLIED CATALYSIS A: GENERAL, ELSEVIER SCIENCE, AMSTERDAM, NL, vol. 214, no. 2, 29 juin 2001 (2001-06-29) , pages 251-257, XP004241020, ISSN: 0926-860X, DOI: DOI:10.1016/S0926-860X(01)00497-5 cité dans la demande
- D. ZHANG ET AL: "One-step dehydration and isomerisation of n-butanol to iso-butene over zeolite catalysts", CHM. COMMUN., vol. 46, 14 avril 2010 (2010-04-14), pages 4088-4090, XP002618573,
- MAKAROVA M A ET AL: "Dehydration of n-butanol on Zeolite H-ZSM-5 and amorphous aluminosilicate: Detailed Mechanistic Study and the Effect of Pore Confinement", JOURNAL OF CATALYSIS, ACADEMIC PRESS, DULUTH, MN, US, vol. 149, 1 janvier 1994 (1994-01-01), pages 36-51, XP002465129, ISSN: 0021-9517, DOI: DOI:10.1006/JCAT.1994.1270 cité dans la demande
- P. BERTEAU ET AL: "Role of the Acid-Base Properties of Alumina, Modified gamma-Alumina, and Silica-Alumina in 1-Butanol Dehydration", APPLIED CATALYSIS, vol. 31, 1987, pages 361-383, XP002618574, cité dans la demande
- R. MANN: "Catalyst deactivation by coke deposition: Approaches based on interactions of coke laydown with pore structure", CATALYSIS TODAY, vol. 37, 1997, pages 331-349, XP002618575, cité dans la demande
- R. TAKAHASHI ET AL: "Effect of diffusion in catalytic dehydration of alcohol over silica-alumina woth continuous macropores", JOURNAL OF CATALYSIS, vol. 229, 2005, pages 24-29, XP002618576,

## Description

### Domaine technique de l'invention

La présente invention concerne un procédé amélioré de production d'alcènes en C4 ou butènes à partir d'une charge de monoalcools en C4 ou butanol. La charge de butanol utilisée est d'origine biologique ou chimique. Ce procédé met en oeuvre un catalyseur amorphe présentant une porosité orientée vers une répartition riche en macro porosité débouchante.

On entend par "macroporosité débouchante" les espaces poreux connectés au réseau poreux et accessibles aux fluides mouillants utilisés dans les méthodes de mesure de porosimétrie telle que la porosimétrie au mercure. Cette macroporosité est donc détectable par cette méthode décrite ci-après.

Les alcènes obtenus, et en particulier l'isobutène et le butène-1, présentent un intérêt important dans le domaine de l'industrie pétrochimique et de la synthèse organique, l'isobutène étant un composé clé dans la chimie des grands intermédiaires.

### Art antérieur

La déshydratation des mono-alcools en C4 a été étudiée depuis de nombreuses années, principalement comme voie de synthèse ou de purification de l'isobutène. Des catalyseurs à base d'acide minéral, de sel FeCl₂, d'oxydes métalliques comme l'alumine ou de zéolithe ont été utilisés sur cette application. On citera l'article de Adkins et al, JACS Avril 1925, vol 47, p 1163-1167, où il est noté que l'alumine, après activation sous air, est efficace pour une courte durée en déshydratation de nombreux alcools. Les auteurs notent que seule l'activation sous air permet de rendre l'alumine active sur cette application.

On citera également l'article de Makarova & al (Journal of catalysis, 149, 36-51 (1994)), où l'on enseigne que les zéolithes (ZSM-5) et la silice alumine sont également actives en déshydratation. En conclusion de leur étude, les auteurs notent l'absence de problèmes diffusionnels sur ces deux systèmes, une activité voisine étant observée sur des systèmes mettant en oeuvre des solides présentant des tailles de pores variant de 0,5 à 5 nm.

On citera enfin les travaux de P.Berteau et al décrits dans Applied Catalysis, volume 31, Issue 2, 1987, p 361-383 qui cherchent à moduler l'acidité de l'alumine par ajout de promoteur (F, Na⁺) en observant l'effet sur la réaction de déshydratation. Le cokage est reporté sur ces solides, mais aucune désactivation notable sur les 35 à 70 heures du test n'est mentionnée. Il est noté que l'ajout de Na⁺ provoque une baisse notable de l'activité du catalyseur aluminique.

La demande de brevet EP-B1-0 659 719 décrit un procédé d'isomérisation du squelette et de la position de la double liaison des oléfines en C4 utilisant une matière à base d'alumine, ayant subi une mise en forme particulière en présence d'un précurseur à base de silice. Cette réaction est utilisée dans un procédé où de l'eau est co-introduite avec la charge oléfinique afin de limiter les réactions parasites. Les tests donnés en exemple indiquent des performances obtenues après 1 h de fonctionnement, ce qui est relativement court et peut laisser penser que la stabilité du catalyseur dans le temps reste perfectible. La réaction d'isomérisation squelettale étant toujours accompagnée de réactions secondaires indésirables, une perte d'activité du catalyseur peut être expliquée par des réactions de cokage.

La désactivation par cokage peut se traduire par un dépôt de coke bloquant l'ensemble des sites actifs ou à l'accumulation de ce dépôt principalement en ouverture de pores condamnant ainsi l'accès à certains sites encore vacants. Les travaux de Mann (Catalysis Today 37 (1997) 331-349) ont bien décrit les interactions entre la structure poreuse des catalyseurs, les dépôts de coke dans ses structures et leurs impacts sur l'activité. Ces travaux ont montré en particulier que les dépôts de coke peuvent occuper une proportion importante du volume des pores et de ce fait commencer à modifier la structure et distribution poreuse du catalyseur. Cette modification peut se traduire par un accès plus difficile des molécules aux sites catalytiques voire empêcher l'accès à certains sites ce qui a impact fort sur la stabilité du catalyseur et fréquemment sur la sélectivité. Pour limiter cette obstruction des pores et laisser accessible le plus de sites possibles, il convient d'augmenter le volume poreux ce qui se fait au détriment du nombre de sites actifs et donc du niveau d'activité.

Les travaux de Macho et al. (Applied Catalysis A : General 214 (2001) 251-257) portent sur la déshydratation et l'isomérisation squelettale et de la position de la double liaison simultanées, sur des alcools en C4. Dans ces travaux, des alumines gamma éventuellement activées à l'acide ont été utilisées avec différents monoalcools comprenant une chaine aliphatique à 4 carbones pour évaluer leur sélectivité en terme de production d'oléfines correspondantes et pertes en autres produits non désirés tels que le méthane, l'éthane, l'ethylène, le propane, le propylène, et les produits C5+. Il est ainsi démontré qu'une alumine fonctionnalisée à l'acide sulfurique est plus active et plus sélective qu'une alumine d'origine commerciale pour l'application combinée visée, à savoir la déshydratation conjuguée à l'isomérisation du squelette et de la position de la double liaison des oléfines résultantes. D'après ce document, il semble donc qu'une alumine la plus acide possible soit nécessaire pour réaliser conjointement la déshydratation et l'isomérisation des monoalcools en C4.

La présente invention se propose de fournir un procédé de production d'alcènes en C4, à partir de monoalcools en C4 dans lequel la durée de cycle, ainsi que l'activité dans le temps du catalyseur utilisé sont améliorées par rapport aux catalyseurs à base d'alumine de l'art antérieur.

### Objet et intérêt de l'invention

La présente invention a pour objet un procédé de production d'oléfines en C4, à partir d'une charge de monoalcool en C4 dans lequel on réalise une réaction de déshydratation du monoalcool en au moins une oléfine, et une réaction d'isomérisation squelettale d'au moins une des oléfines produites dans une même enceinte réactionnelle, en présence d'un catalyseur à base d'alumine à porosité adaptée.

L'alcool est déshydraté et l' (les) oléfine(s) produite est (sont) isomérisée(s). L'isomérisation squelettale est une réaction nettement plus rapide que la réaction de déshydratation, et par conséquent, il n'est pas possible généralement de s'arrêter à la réaction de déshydratation.

Le procédé selon l'invention présente l'avantage d'améliorer la durée de cycle du catalyseur, grâce à la répartition poreuse riche en macroporosité débouchante.
L'activité du catalyseur dans le temps est ainsi améliorée. Cet effet est probablement dû à l'augmentation du volume offert au coke catalytique, tout en minimisant la perte dans le temps de sites actifs disponibles pour la réaction.

Il convient de noter que l'augmentation de volume offert au coke catalytique se fait au détriment du nombre de sites actifs de l'alumine, mais ne provoque pas de baisse sensible d'activité, ce qui est que nature surprenante au vu des enseignements existant sur cette thématique. On se trouve ainsi en disposant d'un catalyseur plus ouvert dans une gamme bien ciblée d'un catalyseur actif et capable d'une longue durabilité sur l'application.

### Description détaillée de l'invention

La présente invention a pour objet un procédé de production d'oléfines en C4, par passage d'une charge de monoalcool en C4 sur un catalyseur, dans lequel on réalise une réaction de déshydratation du monoalcool en au moins une oléfine, et une réaction d'isomérisation squelettale d'au moins une des oléfines produites dans une même enceinte réactionnelle, ledit procédé étant caractérisé en ce que les réactions de déshydratation et d'isomérisation sont mises en oeuvre en présence du catalyseur, contenant éventuellement un promoteur, le dit catalyseur étant à base d'alumine, exempt d'halogènes et présentant une distribution poreuse telle que le volume des pores de diamètre supérieur à 0,1 µm mesuré par porosimétrie au mercure selon la norme ASTM D4284-83 est compris entre 10 mL/100g et 30mL/100g, et le catalyseur comprend au moins 50% poids d'alumine gamma.

Selon la présente invention, la déshydratation des monoalcools en C4 et la réaction d'isomérisation squelettale de l'alcène obtenu après déshydratation, sont catalysées par le même catalyseur, sur des sites de même nature.

Le catalyseur mis en oeuvre dans le procédé selon la présente invention possède la caractéristique d'avoir un volume macroporeux adapté. L'adaptation de ce volume est réalisée par des traitements usuels de l'homme de l'art parmi lesquels on citera l'ajout d'un porogène organique d'origine polymère on non (compatible avec sa destruction lors de la calcination finale du solide), vieillissement à la vapeur, autoclavage, attaque chimique suivie de lavage, précokage.

Le catalyseur utilisé dans le procédé selon l'invention se présente sous forme de sphéroïdes. Ce type de forme, une fois placée dans les réacteurs, présente l'avantage de pouvoir se décharger facilement, sans former de bouchons, même une fois que le catalyseur est coké. Elle présente aussi l'intérêt d'assurer un chargement homogène contrairement aux solides sous forme d'extrudés, ainsi qu'une plus grande résistance mécanique à l'écrasement.

Le catalyseur de forme sphéroïde présente une double porosité mesurée par porosimétrie au mercure. L'analyse de porosimétrie au mercure correspond à l'intrusion d'un volume de mercure caractéristique de l'existence de mésopores et de macropores dans ledit catalyseur selon la norme ASTM D4284-83 à une pression maximale de 4000 bars, utilisant une tension de surface de 484 dynes/cm et un angle de contact de 140° (valeur choisie suivant les recommandations de l'ouvrage "Technique de l'ingénieur, traité analyse et caractérisation", page 1050, écrit par J. Charpin et B. Rasneur), les pores étant supposés de forme cylindrique. Cette technique permet d'accéder à la valeur du volume mercure mésoporeux défini comme étant le volume mercure adsorbé par l'ensemble des pores ayant un diamètre compris dans la gamme des mésopores à savoir compris entre 3,6 et 100 nm. De même, le volume mercure macroporeux est défini comme étant le volume mercure adsorbé par l'ensemble des pores ayant un diamètre supérieur à 100 nm.

La double porosité du catalyseur utilisé dans le procédé selon la présente invention est caractérisée de la façon suivante: une macroporosité caractérisée par un volume mercure macroporeux compris dans une gamme variant de 0,10 à 0,30 ml/g et de préférence dans une gamme variant de 0,12 à 0,25 ml/g et une mésoporosité caractérisée par un volume mercure mésoporeux compris dans une gamme variant de 0,25 à 0,7 ml/g de préférence dans une gamme variant de 0,34 à 0,48 ml/g. La macroporosité est également caractérisée par la présence de domaines macroporeux au delà de 100 nm et/ou résulte d'une macroporosité texturale intraparticulaire, la mésoporosité est aussi caractérisée par la présence de domaines mésoporeux compris dans une gamme variant de 7 à 50 nm et de préférence dans une gamme variant de 8 à 10 nm. La proportion du volume poreux accessible (non occlus) desdites sphéroïdes ayant une taille de pores inférieure à 20 nm est comprise entre 60 et 75 %

Le catalyseur utilisé dans le procédé selon la présente invention présente un volume poreux total défini par l'analyse de porosité mercure précitée compris entre 0,45 et 0,9 ml/g.

Le catalyseur est préparé selon les techniques connues de l'homme du métier. Dans un premier temps l'alumine ayant la macroporosité selon l'invention est préparée, en utilisant un porogène, optionnellement une technique d'ouverture des pores, et enfin une calcination sous air à une température supérieure à 550°C. Le promoteur est ajouté à l'alumine pour la mise en forme ou bien est imprégné après mise en forme.

De préférence, le catalyseur solide est mis en forme par l'une des techniques connues de l'homme de l'art, consistant dans la technique de coagulation en gouttes (encore connu sous l'appellation de méthode « Oil Drop » ou la technique par granulation (dragéification).

La morphologie et la distribution en tailles des billes ainsi obtenues sont établies par analyse de photos obtenues par microscopie électronique à balayage (MEB) et par analyse d'image (caméra, détermination de la sphéricité).

Le catalyseur selon l'invention se présente sous forme de sphéroïdes qui de préférence ont un diamètre moyen compris entre 1 et 2,5 mm. Par le terme « diamètre moyen », on définit le diamètre maximum d'un cercle permettant d'englober la totalité de la bille qu'elle soit ovoïde ou sphérique.

Le catalyseur présente une surface spécifique S_{BET} comprise entre 180 et 270 m²/g.

Le catalyseur mis en oeuvre dans la présente invention est à base d'alumine et comprend au moins 50% poids d'alumine gamma, et de préférence au moins 65% poids, et de manière encore plus préférée au moins 80%pds.

Il peut éventuellement comprendre en outre jusqu'à 35% poids, et de préférence au plus 20% poids,et de manière encore plus préférée entre 0,1 et 35% poids ou entre 0,1 et 20% poids d'un autre oxyde métallique, structuré ou non. De façon préférée, ledit autre oxyde est une zéolite, par exemple ZSM-5.

L'utilisation de sphéroïdes de diamètre contrôlé à un faible diamètre permet de plus de maximiser l'effet constaté, en développant au maximum la surface offerte au coke catalytique.

Au moins un promoteur peut être ajouté au catalyseur, ce qui permet d'ajuster l'activité isomérisante du catalyseur.

Le promoteur est choisi parmi les métaux des groupes 4 (Ti , Zr, Hf), 5 (V, Nb, Ta), 6 (Cr, Mo, W) et/ou 12 (Zr, Cd, Hg) ou les oxydes de métaux alcalins (Na, K).

La présence d'un promoteur des groupes 4, 5, 6 et/ou 12, éventuellement couplée à une augmentation de la température réactionnelle (de préférence température d'au moins 480°C et d'au plus 600°C ou 570°C), permet d'augmenter le taux d'isomérisation squelettale, alors que la présence de promoteur métal alcalin, choisi parmi le potassium et/ou le sodium, oriente peu vers la réaction d'isomérisation squelettale.

Lorsque le promoteur est un élément des groupes 4, 5, 6 et/ ou 12, la quantité de promoteur ajoutée au catalyseur est de préférence d'au moins 0,1% poids (calculé oxyde) et d'au plus 1 % poids par rapport au catalyseur.
Le plus souvent, le promoteur est Ti, V, W. De façon préférée, le promoteur est Ti.
Un catalyseur préféré est constitué d'alumine et d'oxyde du(des) promoteur(s), de préférence le promoteur est Ti, V, W, et de préférence c'est l'oxyde de titane.

Lorsque le promoteur est le sodium ou le potassium (de préférence sous forme oxyde), la quantité de promoteur ajoutée au catalyseur est d'au moins 0,1% pds et d'au plus 2% pds (calculé oxyde). Un catalyseur préféré est constitué d'alumine et d'oxyde du(des) promoteur(s).

Le catalyseur, après calcination, ne contient pas d'halogène.

Le monoalcool en C4 contenu dans la charge peut avoir des sources diverses : en particulier il peut avoir été produit par voie biologique ou par voie chimique.

Le n-butanol peut être ainsi obtenu par fermentation acétonobutylique du glucose, comme décrit dans le brevet FR-B1-2 634 218. On peut citer également la demande de brevet US 2009/226991 qui décrit la production d'isobutanol par voie biologique.

L'alcool peut également être issu d'un procédé de transformation par voie chimique.
Le monoalcool en C4 peut être obtenu à partir de syngas ou par hydratation d'oléfines directe ou indirecte, on par synthèse oxo. L'hydratation des oléfines afin d'obtenir des alcools, dont les alcools en C4, est détaillée dans "Techniques de l'ingénieur, Opérations unitaires. Génie de la réaction chimique, Référence J5550, Date de publication : 10 mars 1997, Bernard TORCK". On se reportera également à l'ouvrage "Procédés de pétrochimie: Les grands intermédiaires oxygénés, chlorés et nitrés" Par Alain Chauvel, Gilles Lefebvre, L. Castex, Pierre Leprince, Ecole nationale supérieure du pétrole et des moteurs (France). Centre d'études supérieures de raffinage et de génie chimique.

Le monoalcool en C4 utilisé dans le procédé est choisi parmi le 1-butanol, le 2-butanol, l'isobutanol, le tert-butanol, seuls ou en mélange.

De façon très préférée, l'alcool en C4 est l'isobutanol

La charge contenant le monoalcool en C4 peut comprendre jusqu'à 50 % poids d'eau, ainsi que les impuretés liés au procédé de production (azote, acides, aldéhydes, alcools non C4 principalement)

De plus, la présence d'eau dans le procédé, qu'elle soit introduite avec l'alcool ou bien qu'elle résulte de la réaction de déshydratation de l'alcool, permet avantageusement de limiter les réactions d'oligomérisation, inévitables lors de la réaction d'isomérisation squelettale.

Le procédé selon la présente invention est opéré à une température comprise entre 250 et 600°C, de préférence entre 330 et 570°C, sous une pression comprise entre à 0,1 et 1 MPa, de préférence entre 0,1 et 0,5 MPa, avec une vitesse spatiale horaire (volume de charge par volume de catalyseur et par heure) comprise entre 0,1 et 10 h⁻¹, de préférence entre 0,8 et 1,5 h⁻¹.

L'unité réactionnelle pourvue du catalyseur sous forme de sphéroïdes employée pour la mise en oeuvre du procédé selon la présente invention fonctionne soit en lit fixe soit en lit mobile, préférentiellement en lit fixe. Lorsqu'elle fonctionne en lit fixe ou en lit mobile, le catalyseur est régénéré périodiquement et ladite unité réalise alternativement la réaction pour la production d'alcènes en C4 et la régénération dudit catalyseur de manière à éliminer le coke déposé à sa surface pendant les réactions. Selon une utilisation alternative, en lit mobile, le catalyseur peut être transporté entre la zone de réaction et la zone de régénération.

### EXEMPLES

### Exemple 1 : synthèse de catalyseur

On réalise la synthèse par dragéification à partir de poudre d'alumine dans une assiette tournante. Les dragées sont séchées et calcinées sous air de façon appropriée à l'obtention d'une alumine gamma. On obtient ainsi le catalyseur A. C'est ce type de catalyseur (alumine activé grade A), qui est le souvent utilisé dans les publications de l'art antérieur). Le catalyseur de référence provient de la société Axens, et est répertorié dans leur catalogue sous les noms AA 2/5 Grade A.
Il s'agit d'un catalyseur de forme sphérique, ayant un diamètre compris entre 2 et 5 nm (voir tableau 1 ci-dessous).

Un post traitement à la vapeur, extraction à l'eau, séchage et calcination est utilisé pour créer des pores plus larges à partir du catalyseur A afin d'obtenir les catalyseurs B et C, ceci au détriment d'une faible perte de surface. De plus, un tamisage est utilisé pour ne prélever que la fraction de plus faible diamètre du catalyseur. Entre les catalyseurs B et C, on fait varier la sévérité du traitement de vieillissement hydrothermal et on ajuste également les étapes de lavage.

Un précurseur organique solide (micro billes de polymère) est utilisé lors de la dragéification de la poudre d'alumine, avant d'utiliser les mêmes étapes que le catalyseur C. On obtient ainsi le catalyseur D dont le volume des pores de diamètre supérieur à 0,1 µm est de 33 mL/100 g et est donc non conforme à l'invention .

Le catalyseur E est obtenu à partir catalyseur C en ajoutant avant le traitement hydrothermal, une imprégnation à sec par une solution d'hydroxyde de sodium.

Le catalyseur F est obtenu par une technique Oil Drop à partir d'un gel d'alumine où l'on a ajouté du TiO₂. Un porogène organique a également été utilisé (hydrocarbure isoparaffinique). La calcination finale est ajustée pour obtenir une macroporosité ciblée.

Le tableau ci dessous détaille les caractérisations des catalyseurs obtenus.

**Tableau 1**

| | **A (comparatif)** | **B** | **C** | **D (comparatif)** | **E** | **F** |
|---|---|---|---|---|---|---|
| **V_{>0,1 µm}(mL/100g)** | 3 | 15 | 20 | 33 | 21 | 17 |
| **Surface (m²/g)** | 335 | 270 | 195 | 142 | 187 | 201 |
| **V_{poreux Total} (mL/100g)** | 37 | 54 | 68 | 104 | 69 | 65 |
| **Diamètre moyen(mm)** | 3,5 | 2,8 | 2,5 | 2,3 | 2,5 | 2,0 |
| **V_{méso}(mL/100g)** | 34 | 39 | 48 | 71 | 48 | 48 |
| **Promoteur** | - | - | - | - | Na2O 1% | TiO2 0.5% |

### Exemple 2 : Activité en déshydratation du 1-butanol

Chaque catalyseur est testé sur environ 500h de façon identique afin de comparer leurs performances en terme de durée de vie et d'activité. On utilise 75 g de catalyseur dilué afin d'avoir le profil thermique le plus isotherme possible, la réaction étudié étant fortement endothermique.

Avant le test proprement dit, on procède à l'activation du solide à 550°C sous air durant 2 heures. Cette activation consiste en une calcination, qui vise à la combustion des traces d'huile, de graisse et au séchage du catalyseur avant son utilisation.

On injecte 75 g/h de butanol-1 pur commercial additivé de 7% d'eau distillée sur ce catalyseur. A la sortie du réacteur, on procède à la séparation de la phase gaz, de la phase liquide organique et de la phase liquide aqueuse. On ne pratique pas de recyclage. Les conditions de réaction mises en jeu sont : une température de 380°C, le réacteur étant isotherme, et une pression de 0,5 bars relatif.

Les analyses des phases aqueuses et organiques gaz indiquent la nature des composés formés. On ne note pas de présence de phase organique liquide à séparer de la phase aqueuse pour l'ensemble des tests.

La partie hydrocarbure de la phase gaz contient majoritairement des butènes du propylène et des pentènes, ainsi que des traces d'hydrocarbures en C1, C2,C3, C5 et C6 et de faibles quantités de CO, de CO₂ et d'hydrogène.
La phase aqueuse contient également d'autres dérivés oxygénés (éthers et cétones principalement du dibutyléther) mais la quantification de ces espèces n'a pas été réalisée. Seule l'eau a été comptabilisée et la teneur en composés non convertis en hydrocarbure est regroupé sous la mention "alcool".

La conversion en alcool est suivie en titrant l'alcool présent dans la phase aqueuse en sortie de l'unité. La masse de phase aqueuse est par contre recalculée à partir de la conversion afin de normaliser à 100 le bilan matière.

Les résultats obtenus avec le catalyseur de référence, selon l'art antérieur sont donnés dans le tableau 2.

**Tableau 2 (comparatif)**

| **Cata A** | ***Charge*** | **effluent** | | | |
|---|---|---|---|---|---|
| **Temps sous charge** | | **1h** | **30h** | **150h** | **350h** |
| Méthane | | 0,09 | 0,09 | 0,08 | 0,07 |
| Éthane | | 0,02 | 0,01 | 0,03 | 0,02 |
| Éthylène | | 0,29 | 0,13 | 0,12 | 0,09 |
| Propane | | 0,00 | 0,00 | 0,00 | 0,00 |
| Propylène | | 0,61 | 0,29 | 0,27 | 0,23 |
| n-butane | | 0,02 | 0,02 | 0,02 | 0,01 |
| Butène-2-trans | | 13,64 | 14,39 | 18,32 | 14,83 |
| butène-1 | | 31,47 | 35,01 | 43,79 | 39,04 |
| isobutène | | 1,04 | 0,77 | 0,00 | 0,00 |
| butène-2-cis | | 19,41 | 14,66 | 1,35 | 1,12 |
| somme C5 | | 1,88 | 1,58 | 0,86 | 0,13 |
| somme C6 | | 1,08 | 0,91 | 0,52 | 0,08 |
| somme C6+ | | 0,17 | 0,14 | 0,07 | 0,01 |
| Eau(recalc) | 5 | 27,4 | 27,0 | 26,0 | 22,8 |
| Alcool | 95 | 3 | 5 | 9 | 22 |
| *Somme* | *100* | *100* | *100* | *100* | *100* |
| Conversion | | 97 | 95 | 91 | 77 |
| Sélectivité 1-butène | | 45 | 51 | 67 | 71 |
| Sélectivité Butène | | 94 | 95 | 97 | 100 |

Les tests réalisés selon le procédé de l'invention mettant en oeuvre les catalyseurs B à E sont donnés respectivement dans les tableaux 3 à 6 suivants :

**Tableau 3**

| **Cata B** | ***Charge*** | **effluent** | | | |
|---|---|---|---|---|---|
| **Temps sous charge** | | **1h** | **30h** | **150h** | **350h** |
| Méthane | | 0,09 | 0,09 | 0,08 | 0,07 |
| Éthane | | 0,02 | 0,01 | 0,03 | 0,02 |
| Éthylène | | 0,27 | 0,13 | 0,12 | 0,09 |
| Propane | | 0,00 | 0,00 | 0,00 | 0,00 |
| Propylène | | 0,67 | 0,29 | 0,27 | 0,23 |
| n-butane | | 0,02 | 0,02 | 0,02 | 0,01 |
| Butène-2-trans | | 13,20 | 18,15 | 18,04 | 17,07 |
| butène-1 | | 42,74 | 43,21 | 46,75 | 47,20 |
| isobutène | | 6,38 | 3,32 | 0,67 | 0,00 |
| butène-2-cis | | 1,47 | 1,80 | 1,33 | 1,29 |
| somme C5 | | 2,27 | 1,17 | 0,62 | 0,23 |
| somme C6 | | 1,30 | 0,68 | 0,38 | 0,13 |
| somme C6+ | | 0,21 | 0,10 | 0,05 | 0,02 |
| Eau(recalc) | *4,3* | 26,4 | 26,5 | 26,2 | 25,6 |
| Alcool | 95,7 | 5 | 4 | 6 | 8 |
| *Somme* | *100* | *100* | *100* | *100* | *100* |
| Conversion | | 95 | 95 | 94 | 91 |
| Sélectivité 1-butène | | 62 | 63 | 69 | 71 |
| Sélectivité Butène | | 93 | 97 | 98 | 99 |

**Tableau 4**

| **Cata C** | ***Charge*** | **effluent** | | | |
|---|---|---|---|---|---|
| **Temps sous charge** | | **1h** | **30h** | **150h** | **350h** |
| Méthane | | 0,09 | 0,09 | 0,08 | 0,07 |
| Éthane | | 0,02 | 0,01 | 0,03 | 0,02 |
| Éthylène | | 0,19 | 0,13 | 0,12 | 0,09 |
| Propane | | 0,00 | 0,00 | 0,00 | 0,00 |
| Propylène | | 0,55 | 0,29 | 0,27 | 0,23 |
| n-butane | | 0,02 | 0,02 | 0,02 | 0,01 |
| Butène-2-trans | | 12,61 | 13,53 | 14,18 | 15,57 |
| butène-1 | | 44,72 | 46,68 | 48,70 | 48,68 |
| isobutène | | 4,41 | 2,56 | 0,65 | 0,00 |
| butène-2-cis | | 1,25 | 1,18 | 1,40 | 1,54 |
| somme C5 | | 1,88 | 1,62 | 0,91 | 0,16 |
| somme C6 | | 1,08 | 0,94 | 0,55 | 0,09 |
| somme C6+ | | 0,17 | 0,14 | 0,08 | 0,01 |
| Eau(recalc) | 5 | 26,5 | 26,7 | 26,5 | 26,3 |
| Alcool | 95 | 6 | 6 | 7 | 8 |
| *Somme* | *100* | *100* | *100* | *100* | *100* |
| Conversion | | 93 | 94 | 93 | 92 |
| Sélectivité 1-butène | | 67 | 69 | 73 | 74 |
| Sélectivité Butène | | 94 | 95 | 97 | 100 |

**Tableau 5 (comparatif)**

| **Cata D** | ***Charge*** | **effluent** | | | |
|---|---|---|---|---|---|
| **Temps sous charge** | | **1h** | **30h** | **150h** | **350h** |
| Méthane | | 0,09 | 0,07 | 0,08 | 0,07 |
| Éthane | | 0,02 | 0,01 | 0,03 | 0,02 |
| Éthylène | | 0,11 | 0,10 | 0,03 | 0,00 |
| Propane | | 0,00 | 0,00 | 0,00 | 0,00 |
| Propylène | | 0,24 | 0,22 | 0,07 | 0,01 |
| n-butane | | 0,02 | 0,02 | 0,02 | 0,01 |
| Butène-2-trans | | 11,38 | 10,88 | 7,01 | 6,39 |
| butène-1 | | 40,05 | 41,77 | 41,40 | 36,25 |
| isobutène | | 0,85 | 0,65 | 0,00 | 0,00 |
| butène-2-cis | | 1,13 | 0,95 | 4,67 | 4,44 |
| somme C5 | | 0,74 | 0,71 | 0,23 | 0,02 |
| somme C6 | | 0,43 | 0,41 | 0,14 | 0,01 |
| somme C6+ | | 0,07 | 0,06 | 0,02 | 0,00 |
| Eau(recalc) | *4,5* | 22,1 | 22,3 | 21,6 | 19,5 |
| Alcool | *95* | 23 | 22 | 25 | 33 |
| *Somme* | *100* | *100* | *100* | *100* | *100* |
| Conversion | | 76 | 77 | 74 | 65 |
| Sélectivité 1-butène | | 73 | 75 | 77 | 77 |
| Sélectivité Butène | | 97 | 97 | 99 | 100 |

**Tableau 6**

| **Cata E** | ***Charge*** | **effluent** | | | |
|---|---|---|---|---|---|
| **Temps sous charge** | | **1h** | **30h** | **150h** | **350h** |
| Méthane | | 0,09 | 0,09 | 0,08 | 0,07 |
| Éthane | | 0,02 | 0,01 | 0,03 | 0,02 |
| Éthylène | | 0,08 | 0,07 | 0,04 | 0,01 |
| Propane | | 0,00 | 0,00 | 0,00 | 0,00 |
| Propylène | | 0,16 | 0,15 | 0,09 | 0,02 |
| n-butane | | 0,02 | 0,02 | 0,02 | 0,01 |
| Butène-2-trans | | 2,58 | 3,12 | 2,49 | 1,64 |
| butène-1 | | 51,07 | 52,56 | 53,36 | 53,63 |
| isobutène | | 0,00 | 0,00 | 0,00 | 0,00 |
| butène-2-cis | | 1,87 | 2,08 | 1,53 | 1,19 |
| somme C5 | | 0,50 | 0,47 | 0,30 | 0,08 |
| somme C6 | | 0,29 | 0,27 | 0,18 | 0,05 |
| somme C6+ | | 0,05 | 0,04 | 0,03 | 0,01 |
| Eau(recalc) | *4,5* | 22,6 | 23,3 | 23,0 | 22,6 |
| Alcool | *95* | 21 | 18 | 19 | 21 |
| *Somme* | *100* | *100* | *100* | *100* | *100* |
| Conversion | | 78 | 81 | 80 | 78 |
| Sélectivité 1-butène | | 90 | 89 | 92 | 95 |
| Sélectivité Butène | | 98 | 98 | 99 | 100 |

Selon ces résultats, on note que :
- la durée de vie du catalyseur est augmentée par la présence de macroporosité importante : la baisse d'activité est plus faible à l'issue des 350 h de cycle ce qui permet d'envisager un cycle plus long pour les catalyseurs mis en oeuvre dans le procédé selon la présente invention.
- La sélectivité vers l'alcool recherché (1-butène pour 1-butanol, isobutène pour isobutanol) est augmentée par l'ajout d'un promoteur (Na2O) ainsi que par la baisse de surface externe,
- La réduction de l'activité initiale est largement compensée par le gain en stabilité, l'activité résiduelle au delà des 350h étant plus élevée.

### Exemple 3 : Activité en déshydratation du 1-butanol avec isomérisation squelettale

Chaque catalyseur est testé sur environ 100h de façon identique afin de comparer leurs performances en terme de durée de vie et d'activité. On utilise 75 g de catalyseur dilué afin d'avoir le profil thermique le plus isotherme possible, la réaction étudiée étant fortement endothermique.

Avant le test proprement dit, on a procédé à l'activation du solide à 550°C sous air durant 2 h. Cette activation consiste en une calcination, qui vise à la combustion des traces d'huile, de graisse et au séchage du catalyseur avant son utilisation.

On injecte 75 g/h de butanol-1 pur commercial additivé d'eau distillé sur ce catalyseur. A la sortie du réacteur, on procède à la séparation de la phase gaz, de la phase liquide organique et de la phase liquide aqueuse. On ne pratique pas de recyclage. Les conditions de réaction mises en jeu sont : une température de 480°C, le réacteur étant isotherme, et une pression de 0,5 bars relatif.

Les analyses des phases aqueuses et organiques gaz indiquent la nature des composés formés. On ne note pas de présence de phase organique liquide à séparer de la phase aqueuse pour l'ensemble des tests.

La partie hydrocarbure de la phase gaz contient majoritairement des butènes du propylène et des butènes, ainsi que des traces d'hydrocarbures en C1, C2,C3, C5 et C6 et de faibles quantités de CO, de CO₂ et d'hydrogène.

La phase aqueuse contient également d'autres dérivées oxygénés (éthers et cétones principalement du dibutyléther) mais la quantification de cette espèce n'a pas été réalisé. Seule l'eau a été comptabilité et la teneur en composés non converti en hydrocarbure est regroupé sous la mention "alcool".

**Tableau 7**

| **Cata A : isom sq** | ***Charge*** | **effluent** | | | |
|---|---|---|---|---|---|
| **Temps sous charge** | | **1h** | **30h** | **60h** | **100h** |
| Méthane | | 0,14 | 0,16 | 0,15 | 0,16 |
| Éthane | | 0,04 | 0,02 | 0,05 | 0,05 |
| Éthylène | | 1,87 | 1,30 | 0,75 | 0,44 |
| Propane | | 0,11 | 0,10 | 0,07 | 0,02 |
| Propylène | | 3,47 | 2,11 | 1,45 | 0,92 |
| n-butane | | 0,02 | 0,02 | 0,02 | 0,01 |
| Butène-2-trans | | 13,12 | 12,46 | 9,20 | 4,84 |
| butène-1 | | 5,87 | 5,18 | 3,40 | 2,27 |
| isobutène | | 25,60 | 30,48 | 36,80 | 34,57 |
| butène-2-cis | | 8,75 | 9,40 | 7,22 | 3,80 |
| somme C5 | | 6,10 | 4,24 | 3,74 | 2,72 |
| somme C6 | | 2,86 | 2,21 | 1,79 | 1,36 |
| somme C6+ | | 3,48 | 2,76 | 2,26 | 1,36 |
| Eau(recalc) | *5,1* | 28,1 | 27,8 | 26,6 | 22,0 |
| Alcool(tert-butanol) | *95* | 0 | 2 | 6 | 25 |
| *Somme* | *100* | *100* | *100* | *100* | *100* |
| Conversion | | 100 | 98 | 93 | 73 |
| Sélectivité 1-butène | | 8 | 7 | 5 | 4 |
| Sélectivité Butène | | 75 | 82 | 85 | 87 |

**Tableau 8**

| **Cata G: isom sq** | ***Charge*** | **effluent** | | | |
|---|---|---|---|---|---|
| **Temps sous charge** | | **1h** | **30h** | **60h** | **100h** |
| Methane | | 0,17 | 0,20 | 0,16 | 0,17 |
| Ethane | | 0,05 | 0,06 | 0,05 | 0,06 |
| Ethylene | | 1,41 | 1,05 | 0,70 | 0,30 |
| Propane | | 0,07 | 0,05 | 0,05 | 0,03 |
| Propylene | | 2,12 | 1,64 | 1,20 | 0,57 |
| n-butane | | 0,02 | 0,02 | 0,02 | 0,01 |
| Butene-2-trans | | 14,93 | 14,60 | 12,77 | 13,07 |
| butene-1 | | 6,36 | 6,04 | 5,56 | 4,36 |
| isobutene | | 26,62 | 30,79 | 34,62 | 36,10 |
| butene-2-cis | | 9,95 | 8,95 | 8,87 | 8,71 |
| somme C5 | | 5,12 | 4,01 | 2,90 | 1,38 |
| somme C6 | | 2,61 | 1,93 | 1,40 | 0,72 |
| somme C6+ | | 2,31 | 1,78 | 1,28 | 0,66 |
| Eau(recalc) | *4,5* | 27,4 | 27,2 | 26,7 | 25,6 |
| Alcool(tert-butanol) | *95* | 1 | 2 | 4 | 8 |
| *Somme* | *100* | *100* | *100* | *100* | *100* |
| Conversion | | 99 | 98 | 96 | 91 |
| Sélectivité 1-butène | | 9 | 9 | 8 | 7 |
| Selectivité Butène | | 81 | 85 | 89 | 95 |

Les tableaux 8 et 9 ci-dessus donnent les différences d'activité catalytiques entre le catalyseur A, selon l'art antérieur et le catalyseur F, imprégné au Ti. On note une conversion de l'alcool de charge supérieure au bout de 100h pour le catalyseur F utilisé dans le procédé selon l'invention. De plus le taux de récupération d'oléfines en C4 (c'est à dire les récupération sélectives d'oléfines à 4 atomes de carbone est globalement augmenté par la présence du profil de porosité du catalyseur utilisé dans le procédé selon la présente invention.

## Revendications

1. Procédé de production d'oléfines en C4, par passage d'une charge de monoalcool en C4 sur un catalyseur, dans lequel on réalise une réaction de déshydratation du monoalcool en au moins une oléfine, et une réaction d'isomérisation squelettale d'au moins une des oléfines produites dans une même enceinte réactionnelle, ledit procédé étant **caractérisé en ce que** les réactions de déshydratation et d'isomérisation sont mises en oeuvre en présence du catalyseur, contenant éventuellement un promoteur, le dit catalyseur étant à base d'alumine, exempt d'halogènes et présentant une distribution poreuse telle que le volume des pores de diamètre supérieur à 0,1 µm mesuré par porosimétrie au mercure selon la norme ASTM D4284-83 est compris entre 10 mL/100g et 30 mL/100g, et le catalyseur comprend au moins 50% poids d'alumine gamma.

2. Procédé selon la revendication 1 dans lequel ledit catalyseur se présente sous forme de sphéroïdes, de préférence ayant un diamètre moyen compris entre 1 et 2,5 mm.

3. Procédé selon l'une des revendications précédentes dans lequel ledit catalyseur a une surface S_{BET} comprise entre 180 et 270 m²/g.

4. Procédé selon l'une des revendications précédentes ledit catalyseur a un volume poreux total défini par analyse de porosimétrie mercure est compris entre 0,45 et 0,9 ml/g

5. Procédé selon l'une des revendications précédentes dans lequel un promoteur choisi dans le groupe formé par les métaux des groupes 4, 5, 6 et/ou 12 est ajouté audit catalyseur.

6. Procédé selon la revendication 5 dans lequel le promoteur est choisi dans le groupe formé par Ti, V,W.

7. Procédé selon l'une des revendications 5 ou 6 dans lequel la quantité de promoteur est d'au moins 0,1% pds et d'au plus 1% pds (calculé oxyde) par rapport au catalyseur.

8. Procédé selon l'une des revendications 1 à 4 dans lequel un promoteur choisi dans le groupe formé par le potassium et/ou le sodium est ajouté audit catalyseur.

9. Procédé selon la revendication 8 dans lequel la quantité de promoteur est d'au moins 0,1% pds et d'au plus 2% pds (calculé oxyde) par rapport au catalyseur.

10. Procédé selon l'une des revendications précédentes dans lequel le monoalcool en C4 contenu dans la charge est choisi parmi le 1-butanol, le 2-butanol, l'isobutanol, le tert-butanol, seuls ou en mélange.

11. Procédé selon l'une des revendications précédentes dans lequel le monoalcool est l'isobutanol

12. Procédé selon l'une des revendications précédentes dans lequel ladite charge comprend jusqu'à 50% pds d'eau.

13. Procédé selon l'une des revendications précédentes **caractérisé en ce qu'**il est opéré à une température comprise entre 250 et 600°C, de préférence entre 330 et 570°C, sous une pression comprise entre à 0,1 et 1 MPa, de préférence entre 0,1 et 0,5 MPa, avec une vitesse spatiale horaire (volume de charge par volume de catalyseur et par heure) comprise entre 0,1 et 10 h⁻¹, de préférence entre 0,8 et 1,5 h⁻¹.

## Patentansprüche

1. Verfahren zur Herstellung von C₄-Olefinen, indem eine C₄-Monoalkoholcharge über einen Katalysator geleitet wird, wobei eine Reaktion der Dehydrierung des Monoalkohols zu mindestens einem Olefin sowie eine Reaktion der Gerüstisomerisierung mindestens eines der erzeugten Olefine innerhalb ein- und derselben Reaktionskammer durchgeführt werden, wobei das Verfahren **dadurch gekennzeichnet ist, dass** die Dehydrierungs- und Isomerisierungsreaktionen in Gegenwart des Katalysators durchgeführt werden, welcher möglicherweise einen Promotor enthält, wobei der Katalysator auf Aluminiumoxid basiert, frei von Halogenen ist und eine Porenverteilung aufweist, die derart ist, dass das Volumen der Poren mit einem Durchmesser von mehr als 0,1 µm, bei einer Messung mittels Quecksilberporosimetrie gemäß der Norm ASTM D4284-83, im Bereich von 10 mL/100 g bis 30 mL/100 g liegt, und der Katalysator mindestens 50 Gewichts-% an gamma-Aluminiumoxid aufweist.

2. Verfahren nach Anspruch 1, wobei der Katalysator in Form kugelartiger Partikel vorliegt, die vorzugsweise einen mittleren Durchmesser im Bereich von 1 bis 2,5 mm aufweisen.

3. Verfahren nach einem der vorhergehenden Ansprüche, wobei der Katalysator eine S_{BET}-Oberfläche im Bereich von 180 bis 270 m²/g aufweist.

4. Verfahren nach einem der vorhergehenden Ansprüche, wobei der Katalysator, bei einer Feststellung mittels quecksilberporosimetrischer Untersuchung, ein Gesamtporenvolumen im Bereich von 0,45 bis 0,9 ml/g hat.

5. Verfahren nach einem der vorhergehenden Ansprüche, wobei dem Katalysator ein Promotor zugesetzt ist, der aus Gruppe ausgewählt ist, welche von den Metallen der Gruppen 4, 5, 6 und/oder 12 gebildet wird.

6. Verfahren nach Anspruch 5, wobei der Promotor aus der Gruppe ausgewählt ist, die von Ti, V, W gebildet wird.

7. Verfahren nach einem der Ansprüche 5 oder 6, wobei die Menge an Promotor mindestens 0,1 Gew.-% und höchstens 1 Gew.-% (berechnet als Oxid) im Verhältnis zum Katalysator beträgt.

8. Verfahren nach einem der Ansprüche 1 bis 4, wobei dem Katalysator ein Promotor zugesetzt ist, der aus Gruppe ausgewählt ist, welche von Kalium und/oder Natrium gebildet wird.

9. Verfahren nach Anspruch 8, wobei die Menge an Promotor mindestens 0,1 Gew.-% und höchstens 2 Gew.-% (berechnet als Oxid) im Verhältnis zum Katalysator beträgt.

10. Verfahren nach einem der vorhergehenden Ansprüche, wobei der C₄-Monoalkohol, der in der Charge enthalten ist, aus 1-Butanol, 2-Butanol, Isobutanol, tert.-Butanol ausgewählt ist, jeweils allein oder in Mischung.

11. Verfahren nach einem der vorhergehenden Ansprüche, wobei es sich bei dem Monoalkohol um Isobutanol handelt.

12. Verfahren nach einem der vorhergehenden Ansprüche, wobei die Charge bis zu 50 Gew.-% an Wasser umfasst.

13. Verfahren nach einem der vorhergehenden Ansprüche, **dadurch gekennzeichnet, dass** es bei einer Temperatur im Bereich von 250 bis 600 °C, vorzugsweise von 330 bis 570 °C, unter einem Druck im Bereich von 0,1 bis 1 MPa, vorzugsweise von 0,1 bis 0,5 MPa, mit einer stundenbezogenen Raumgeschwindigkeit (Chargenvolumen pro Katalysatorvolumen und pro Stunde) im Bereich von 0,1 bis 10 h⁻¹, vorzugsweise von 0,8 bis 1,5 h⁻¹, betrieben wird.

## Claims

1. Method of producing C4 olefins, by passing a feed of C4 monohydric alcohol over a catalyst, in which a reaction of dehydration of the monohydric alcohol to at least one olefin, and a reaction of skeletal isomerization of at least one of the olefins produced in one and the same reaction vessel, are carried out, said method being **characterized in that** the reactions of dehydration and of isomerization are carried out in the presence of the catalyst, optionally containing a promoter, said catalyst being based on alumina, free from halogens and having a pore distribution such that the volume of pores with diameter greater than 0.1 µm measured by mercury porosimetry according to standard ASTM D4284-83 is comprised between 10mL/100g and 30 mL/100g, and the catalyst comprises at least 50 wt.% of gamma alumina.

2. Method according to claim 1 , in which said catalyst is in the form of spheroids, preferably having an average diameter comprised between 1 and 2.5 mm.

3. Method according to one of the preceding claims , in which said catalyst has a surface area S_{BET} comprised between 180 and 270 m²/g.

4. Method according to one of the preceding claims , in which said catalyst has a total pore volume defined by analysis using mercury porosimetry comprised between 0.45 and 0.9 ml/g

5. Method according to one of the preceding claims , in which a promoter selected from the group comprising metals of groups 4, 5, 6 and/or 12 is added to said catalyst.

6. Method according to claim 5, in which the promoter is selected from the group comprising Ti, V, W.

7. Method according to one of claims 5 or 6, in which the quantity of promoter is at least 0.1 wt.% and at most 1 wt.% (calculated as oxide) relative to the catalyst.

8. Method according to one of claims 1 to 4 , in which a promoter selected from the group comprising potassium and/or sodium is added to said catalyst.

9. Method according to claim 8, in which the quantity of promoter is at least 0.1 wt.% and at most 2 wt.% (calculated as oxide) relative to the catalyst.

10. Method according to one of the preceding claims, in which the C4 monohydric alcohol contained in the feed is selected from 1-butanol, 2-butanol, isobutanol, tert-butanol, alone or mixed.

11. Method according to one of the preceding claims, in which the monohydric alcohol is isobutanol.

12. Method according to one of the preceding claims, in which said feed comprises up to 50 wt.% of water.

13. Method according to one of the preceding claims, **characterized in that** it is carried out at a temperature comprised between 250 and 600°C, preferably between 330 and 570 °C, at a pressure comprised between 0.1 and 1 MPa, preferably between 0.1 and 0.5 MPa, with an hourly space velocity (volume of feed per volume of catalyst per hour) comprised between 0.1 and 10 h⁻¹, preferably between 0.8 and 1.5 h⁻¹.
